# EUROPÄISCHE PATENTSCHRIFT

(11) **EP 1 333 781 B1**
(45) Veröffentlichungstag und Bekanntmachung des Hinweises auf die Patenterteilung: **29.12.2004**
(21) Anmeldenummer: 01982033.1
(22) Anmeldetag: 16.11.2001
(51) Int. Cl.: A61F 2/40

(54) **GELENKPROTHESE**
JOINT PROSTHESIS
PROTHESE D'ARTICULATION

(30) Priorität: 16.11.2000 CH 223500
(43) Veröffentlichungstag der Anmeldung: 13.08.2003
(73) Patentinhaber: Horber, Willi, 8005 Zürich (CH)
(72) Erfinder: Horber, Willi, 8005 Zürich (CH)
(74) Vertreter: Walder, Martin Bernhard
(86) Internationale Anmeldenummer: PCT/CH2001/000674
(87) Internationale Veröffentlichungsnummer: WO 2002/039931

(56) Entgegenhaltungen:
- EP-A- 0 024 442
- EP-A- 0 208 578
- EP-A- 0 351 545
- EP-A- 0 586 335
- EP-A- 0 663 193
- EP-A- 0 679 375
- EP-A- 0 712 617
- EP-A- 0 884 032
- EP-A- 0 963 741
- DE-A- 19 616 059
- DE-C- 19 509 037
- DE-U- 29 918 589
- FR-A- 2 773 469
- US-A- 4 318 190
- US-A- 4 528 702

## Beschreibung

### Technisches Gebiet der Erfindung

Die Erfindung betrifft eine Gelenkprothese, insbesondere eine Humeruskopfprothese, gemäss dem Oberbegriff des Anspruchs 1.

### Stand der Technik

Aus der EP 0 024 442 ist eine Verbund-Endoprothese mit einem Metallschaft und einem keramischen Gelenkteil bekannt. Das keramische Teil besitzt eine konische oder zylindrische Ausnehmung. An einem in die Ausnehmung einzuführenden metallischen Zapfen des Metallschaftes sind Tragrippen ausgebildet. Diese Tragrippen werden beim Aufsetzen des härteren keramischen Gelenkteils plastisch und elastisch verformt. Dadurch wird ein Presssitz zwischen den Endoprothesenteilen erreicht

Aus der EP-A- 0 712 617 ist eine Humeruskopfprothese bekannt, bei der eine über einen Stiel mit einer Kopfkalotte verbundene Anlenkkugel in einer Grube mit hohlkugeligem Grund an einem Schaftteil angelenkt ist. Zur Fixierung der Anlenkkugel in der Grube sind eine oder mehrere Madenschrauben vorgesehen, die durch das Schaftteil hindurch gegen die Anlenkkugel geschraubt werden können. In einem Ausführungsbeispiel wird mit der Madenschraube die c-förmig aufgeschnittene Anlenkkugel zusammengepresst, um den in einer zentralen Bohrung in der Anlenkkugel steckenden Stiel darin festzuklemmen. Es ist in einem anderen Ausführungsbeispiel eine Madenschraube vorgesehen, welche entlang der Stielachse durch die Anlenkkugel und gegen den Grubengrund geschraubt werden kann. Mit dieser Schraube wird die Anlenkkugel gegen die Öffnung der Grube gepresst, durch die der Stiel aus der Grube vorsteht In einem weiteren Beispiel sind zur Indexierung der Stellung der Anlenkkugel in der Grube mit Vertiefungen in der Kugeloberfläche zusammenwirkende Noppen am Grubengrund vorgesehen.

Aus der WO 99/34756, die der FR-A-2 773 469 entspricht, ist eine Gelenkprothese gemäß dem Oberbegriff von Anspruch 1 bekannt, bei welcher in einer halbkugeligen Vertiefung im Schaftteil ein darin kugelgelenkartig verschwenkbares Halsteil angelenkt ist Das Halsteil besitzt eine halbkugelige Anlenkfläche und eine gegenüber einer Achse durch derer Kugelmittelpunkt exzentrische Konusfläche zum Aufstecken einer Gelenkkalotte. Das Halsteil weist eine von der Kalottenseite her offene Ausbohrung mit einem halbkugeligen Grund. Mit dem Grund wirkt eine in die Ausbohrung eingeführte, durch eine Öffnung im Grund der Ausbohrung hindurch in das Schaftteil einschraubbare Schraube mit Kugelkopf. Die Kugeloberflächen der halbkugeligen Vertiefung im Schaftteil, der Anlenkfläche am Schaftteil, des Grundes der Ausbohrung und des Schraubenkopfes müssen den gleichen Mittelpunkt aufweisen. Die jeweils zwei zusammenwirkenden Hohl- und Vollkugeloberflächen müssen zudem sehr exakt hergestellt und den gleichen Radius aufweisen. Kleinste Abweichungen von den Idealabmessungen führen dazu, dass das Halsteil mit dem Schaftteil nicht genügend fest verbunden werden kann, um eine unbeabsichtigte Verschwenkung des Halsteils gegenüber dem Schaftteil während des Gebrauchs des Gelenks sicher zu verhindern. Die dazu erforderliche Präzision der Kugeloberflächen erweist sich jedoch als sehr schwierig zu erreichen.

Die DE-U-299 18 589 stellt sich zur Aufgabe, genau diesen Nachteil von zusammenwirkenden Kugeloberflächen zu vermeiden. Sie schlägt dazu eine Endoprothese für eine Schulter vor, bei der in einem Schaftteil ein Drehstück angeordnet ist, das lediglich um eine erste Achse drehbar ist An diesem Drehstück ist ein Richtstück um lediglich eine zweite Achse verschwenkbar angelenkt Auf dem Richtstück kann eine Kopfkalotte angeordnet werden. Das Richtstück erstreckt sich entlang einer Halsachse, die - dank dem sich die erste und die zweite Achse queren - ähnlich wie bei einem Kugelgelenk in jede gewünschte Lage gebracht und darin festgestellt werden kann.

Die DE-U-29918 589 lehrt damit, ein Kugelgelenk zu meiden und die Kugelgelenkbewegung dadurch nachzubilden, dass sie in zwei unabhängige Bewegungen um zwei diskrete Achsen aufgeteilt wird. Die Anlenk- und Reibungsflächen zwischen Schaftteil, Drehstück und Richtstück sind dank der Vermeidung von Kugelflächen Oberflächen von Rotationskörpern wie Zylinder, Torus und Konus. Diese Oberflächen sind einfacher genügend präzise herstellbar.

Zum Feststellen der Schwenkbewegung des Richtstückes gegenüber dem Drehstück wird vorgeschlagen, dass sich die zusammenwirkenden Anlenkflächen vorteilhaft lediglich derart entsprechen, dass eine Linienberührung zwischen ihnen stattfindet. Dazu kann die Anlenkfläche am Drehstück als Rinne mit zwei in einem Winkel zueinander stehenden ebenen Flächen ausgebildet sein. Es kann auch eine Bohrung im Richtstück, an der der Kugelkopf einer Schraube anpresst, konisch ausgeführt sein. Es wird weiter angeregt, dass die Anlenkflächen auch Kanten und Dome aufweisen können, die beim Festziehen der Schraube in die Gegenfläche einpressbar sind. Ein Beispiel dazu ist nicht wiedergegeben.

### Aufgabe der Erfindung

Es ist Aufgabe der vorliegenden Erfindung, bei einer Gelenkprothese mit einem im Knochen verankerbaren Schaftteil und einem kugelgelenkig daran angelenkten Halsteil und einer auf dem Halsteil angeordneten Kopfkalotte, sowohl die Fertigungstoleranzen für die am Kugelgelenk beteiligten Anlenkflächen als auch die Zuverlässigkeit der Immobilisierung des Kugelgelenks gegenüber dem Stand der Technik zu erhöhen.

### Beschreibung der Erfindung

Erfindungsgemäss weist bei einer Gelenkprothese gemäss dem Oberbegriff des Patentanspruchs 1 eine erste der beiden zusammenwirkenden Anlenkflächen wenigstens eine Kante und/ oder Spitze auf. Zudem sind die Anlenkflächen bezüglich Form derart ausgebildet, dass sich lediglich ein oder mehrere Berührungsbereiche zwischen der Kante und/oder Spitze der ersten Anlenkfläche und der auf einer virtuellen Kugeloberfläche liegenden zweiten Anlenkfläche ergeben. Ferner sind die Anlenkflächen bezüglich Material derart ausgebildet, dass unter Einwirkung der beim Anpressen auftretenden Druckkräfte wenigstens die zweite Anlenkfläche im Berührungsbereich durch die Kante und/oder Spitze der ersten Anlenkfläche plastisch verformbar ist.

Dadurch wird beim Anpressen des Pressteils wenigstens eine Kante oder Spitze der einen Anlenkfläche in die zweite Anlenkfläche eingegraben und so eine Verzahnung oder Verkrallung der beiden Anlenkflächen erreicht Die Verzahnung erlaubt die Toleranzen bei der Fertigung zu erhöhen. Es wird daher trotz nicht übereinstimmender Flächenform und trotz relativ grosser Massungenauigkeiten ein Kugelgelenk geschaffen, das zuverlässig immobilisiert werden kann. Für eine Verschwenkung oder Verdrehung des einmal befestigten Anlenkkopfes gegenüber der Anlenkmulde ist eine Verformung des Materials und sind daher sehr hohe Kräfte erforderlich. Dennoch ist der Anlenkkopf in der Anlenkmulde geführt und kugelgelenkig verschwenkbar, solange das Pressteil nicht fest angepresst ist, sondern lediglich anliegt.

Die Gelenkprothese weist ein im Knochen verankerbares Schaftteil auf. Dieses ist in bekannter Art auf den Knochen abgestimmt und wird dazu allenfalls aus einem Set von Schaftteilen ausgewählt. Am Schaftteil ist ein Halsteil angelenkt, welches eine Halsachse definiert. Das Halsteil ist zur Aufnahme einer Kopfkalotte ausgerüstet oder Teil der Kopfkalotte. Weiter ist die Gelenkprothese auch mit einer Kopfkalotte bestückt, welche auf die mit der Kopfkalotte zusammenwirkenden Gelenkpfanne ausgelegt ist. Die Kopfkalotte ist möglichst an die entfernte natürliche Kalotte angenähert geformt und wird deshalb allenfalls auch aus einem Set von Kalotten ausgewählt. Die Gelenkprothese weist ferner wenigstens ein Pressteil zum Anpressen des Halsteils an das Schaftteil und Mitteln zum Verbinden von Pressteil und Schaftteil auf. Für die Anlenkung des Halsteils am Schaftteil ist entweder am Halsteils oder am Schaftteil eine Anlenkmulde und am andern Teil ein in der Anlenkmulde anzuordnender Anlenkkopf ausgebildet der Anlenkkopf ist in der Anlenkmulde wenigstens um zwei senkrecht zueinander stehende Achsen verschwenkbar bzw. verdrehbar. Je nachdem, ob die Gelenkpfanne auch ersetzt wird oder nicht, weist die Gelenkprothese auch eine künstliche im Knochen verankerbare Gelenkpfanne auf. Bleibt die natürliche Gelenkpfanne erhalten, wirkt die Kopfkalotte mit dieser zusammen.

Die plastische Verformung eines Berührungsbereichs in der Anlenkmulde oder eines Berührungsbereichs an der Anlenkfläche des Anlenkkopfes führt nicht nur dazu, dass die notwendige Präzision der Teile innerhalb herkömmlicher Herstellungstoleranzen liegen kann und keine erhöhten Anforderungen an die Herstellung und die Überprüfung der Masslialtigkeit der Teile stellt. Die Verformung der Anlenkfläche führt auch zu einer unverschwenkbaren Verbindung zwischen Schaftteil und Halsteil. Im verformten Bereich können bei einer Verpressung der Anlenkflächen beide oder auch nur eine beteiligte Fläche verformt werden. Ist es vorwiegend das Halsteil, welches die Verformung erfährt, kann dieses, z.B. bei einer Revision der Gelenkprothese, erneuert werden, ohne dass das Schaftteil ausgewechselt werden muss.

Weisen die Berührungsbereiche zwischen dem Grund der Anlenkmulde und dem Anlenkkopf eine oder mehrere punktuelle Berührungen auf, oder auch eine oder mehrere durchgehende oder unterbrochene Linienberührungen, so ist in diesen Bereichen die Höhe des ausgeübten Drucks umgekehrtproportional zur Grösse der Berührungsbereiche. Dadurch können stärkere Verformungen erreicht werden.

Ist eine der Anlenkflächen durch eine oder mehrere Körperkanten oder eine oder mehrere Körperspitzen, und die andere Anlenkfläche durch einen Flächenbereich gebildet, so werden die Körperspitzen und Körperkanten in die andere Anlenkfläche eingepresst und es entstehen verformte Anlenkflächen, deren Ausrichtung quer zu einer Tangente zu einem Kreis um das Verschwenkzentrum stehen. Dies bewirkt eine Verzahnung zwischen Halsteil und Schaftteil, und daher eine sichere Verbindung, die auch relativ grossen Kräften auf das Kugelgelenk zwischen Grund und Halsteil widersteht. Der Flächenbereich kann dabei glatt oder rauh sein. Die Möglichkeiten reichen bei glatt von unbehandelt nach spanabhebender Bearbeitung bis poliert Eine rauhe Oberfläche kann gerillt oder porös sein, sie kann geätzt, gestrahlt, gegossen oder spanabhebend bearbeitet oder sonstwie strukturierten sein. Vorteilhaft ist dabei zu berücksichtigen, dass die beiden Anlenkflächen praktisch stufenlos gegeneinander verschwenkbar bleiben. Daher eignen sich rauhe Flächen eher für Linienberührung und glatte eher für Punktberührung.

In den Ausführungsbeispielen liegt der Berührungsbereich auf einer virtuellen Kugeloberfläche. Dies hat den Vorteil, dass die kugelgelenkartige Verschwenkbarkeit zwischen Halsteil und Schaftteil ohne zusätzliche Massnahmen gegeben ist Ist die eine Anlenkfläche, z.B. des Anlenkkopfes, eine Kugeloberfläche, so bildet eine mit diesem Kugeloberflächenbereich zusammenwirkende Anlenkfläche, vorteilhaft Kanten, welche quer zum stärksten der vor und nach der Verklemmung auf das Halsteil einwirkenden Drehmomente liegen. Dies verhindert die Verdrehung des Halsteils um die Halsachse, was sicherstellt, dass die gewählte Stellung zwischen Halsteil und Schaftteil beim oder nach dem Zusammenpressen dieser beiden nicht verändert wird. Es kann aber auch umgekehrt der Kugeloberflächenbereich Rillen und Kanten aufweisen. Diese stehen ebenfalls vorteilhaft quer zum stärksten auf das Halsteil wirkenden Drehmoment Zusätzlich zu den kleinflächigen Berührungsbereichen zwischen Schaftteil und Halsteil ist es auch vorteilhaft solche zwischen Halsteil und Pressteil vorzusehen. Es besteht deshalb mit Vorteil wenigstens eine durchgehende oder unterbrochene Linienberührung zwischen Anlenkkopf und Pressteil.

### Kurzbeschreibung der Figuren

Die Erfindung wird nun anhand von Ausführungsbeispielen erläutert. Es zeigt:
- Fig. 1: eine teilweise geschnittene Darstellung einer erfindungsgemässen Schultergelenkprothese mit kugelförmigem Anlenkkopf in einer Anlenkmulde mit einer Kreiskante als Anlenkfläche,
- Fig. 2: eine teilweise Geschnittenen Darstellung einer Schultergelenkprothese ohne Kopfkalotte mit gegen einen kugelförmigen Grund der Anlenkmulde gerichteten Spitzen am Anlenkkopf des Halsteils,
- Fig. 3: eine teilweise geschnittene Darstellung einer Schultergelenkprothese mit kugelförmigen Anlenkkopf und sowohl in den Anlenkkopf einpressbaren Kreiskanten als auch einer solchen Spitze im Grund der Anlenkmulde,
- Fig. 4: eine teilweise geschnittene Darstellung des Kopfbereichs einer Schultergelenkprothese mit zylindrischem Anlenkkopf in kugeliger Anlenkmulde,
- Fig. 5: eine teilweise geschnittene Darstellung des Kopfbereichs einer Schultergelenkprothese mit am Grund der Anlenkmulde ausgebildeten Kreiskanten in Berührung mit einer Kugeloberfläche am Anlenkkopf und einer unterbrochenen Kreislinie als Anlenkfläche zwischen auf einem Kreis liegenden Spitzen am Anlenkkopf und einer Kugeloberfläche am Pressteil,
- Fig. 6: eine Schnittdarstellung durch den Kopfbereich einer Schulterprothese mit kugelförmigem Anlenkkopf und kegelförmig ausgebildetem Grund der Anlenkmulde,
- Fig. 7: eine teilweise geschnittene Darstellung des Kopfbereiches einer Schulterprothese mit halbkugeligem Grund der Anlenkmulde und darin einem zu diesem Grund hin zylindrisch geformten Anlenkkopf, welcher zur Anpressscheibe mit einer Kreiskante als Anlenkfläche hin eine Kugeloberfläche aufweist,
- Fig. 8: eine Schnittdarstellung durch den Kopfbereich einer Schulterprothese mit halbkugeliger Anlenkmulde und zylindrischem Anlenkkopf, bei welchem das Pressmittel eine Schraube durch das Zentrum des Anlenkkopfes ist,
- Fig. 9 und 10: Ansichten von zwei Teilen, die zusammen ein Halsteil bilden,
- Fig.11: eine Aufsicht auf das Halsteil aus den Teilen gemäss Figuren 9 und 10 mit Exzenterring
- Fig. 12: eine teilweise geschnittene Darstellung einer Schultergelenkprothese mit einem Halsteil gemäss Figuren 9 bis 11 und einem Spannbügel.

### Beschreibung der Ausführungsbeispiele

Der Übersichtlichkeit halber werden in der folgenden detailierten Beschreibung der Ausführungsbeispiele für gleiche und ähnliche Teile die gleichen Bezugsziffern verwendet

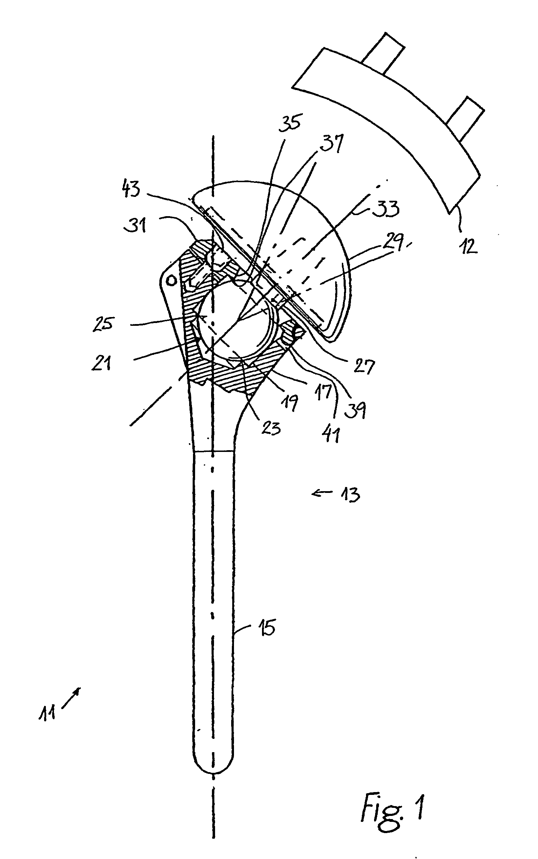

Die in den Figuren 1 bis 9 und 13 dargestellten Schultergelenkprothesen 11 umfassen ein im Humerusknochen eines Patienten verankerbares Schaftteil oder Schaftteil 13 mit Schaftstiel 15 und Schaftkopf 17. Im Schaftkopf 17 ist eine Anlenkmulde 19 ausgebildet. Am Schaftteil 13 ist ein Halsteil 21 angelenkt. Dieses weist einen in der Anlenkmulde 19 aufliegenden Anlenkkopf 25 und daran einen Halsfortsatz 27 auf. Auf den Halsfortsatz 27 ist eine Kopfkalotte 29 aufgesetzt oder aufsetzbar, welche mit einem künstlichen Glenoid 12, wie in Fig. 1 dargestellt, oder mit einem natürlichen Glenoid zusammenwirkt.

Eine Halsachse 33 ist durch die Richtung des Halsfortsatzes 27 definiert. In einer Mittelstellung des Halsteils 21 fällt die Halsachse 33 mit der Achse der Anlenkmulde 19 zusammen. Diese Richtung der Halsachse 31 muss bei jedem Patienten individuell eingestellt werden. Um die Halsachse 33 bezüglich Inklination und Ante- bzw. Retroversion, z.B. senkrecht auf die Schnittfläche am Knochen des Patienten, ausrichten zu können, ist das Halsteil 21 in der Anlenkmulde 19 kugelgelenkartig gelagert Die Halsachse ist so um einen Winkel von ca. 20 Grad in jede Richtung aus der Mittelstellung auslenkbar.

In Figur 1 ist ein erstes Ausführungsbeispiel einer Schultergelenkprothese dargestellt, bei welchem eine Kreiskante 23 am Grund der Anlenkmulde 19 ein Auflager für die Kugel des Anlenkkopfes 25 bildet Diese Kreiskante 23 ist gebildet durch die Mündungskante einer zentralen zweiten Bohrung im ebenen Grund einer das Hauptvolumen der Anlenkmulde 19 bildenden ersten Bohrung im Schaftkopf 17. Die grössere erste Bohrung weist einen dem Kugelradius des Anlenkkopfes 21 entsprechenden Radius auf, kann aber auch grösser sein, um das Einsetzen des Anlenkkopfes 25 in die Anlenkmulde 19 zu erreichen. Die kleinere zweite Bohrung weist einen etwa der Kreiskante 23 entsprechenden Radius auf. Eine Führung der Anlenkkugel 25 wird durch die beiden Kreiskanten 23,37 der kleineren Bohrung und der Durchtrittöffnung 35 erreicht.

In der Anpressscheibe 31 ist eine Durchtrittöffnung 35 für den Halsfortsatz 27 ausgebildet. Ihr Durchmesser kann dem der zweiten Bohrung entsprechen. Die Durchtrittöffnung 35 weist eine kreisrunde Öffnungskante 37 auf, welche am Anlenkkopf 25 anliegt. Auf der einen Seite ist die Anpressscheibe 31 mit einer Scharniernase 39 in einer Scharnierausnehmung 41 im Schaftkopf 17 angelenkt Scharniernase 39 und Scharnierausnehmung 41 sind ineinandersteckbar, so dass Anpressscheibe 31 und Schaftteil 13 voneinander lösbar sind. Auf der Scharnierseite kann zwischen zwei Scharniernasen 39 eine Schlitzöffnung in der Anpressscheibe 31 ausgebildet sein, so dass die Anpressscheibe 31 hufeisen- oder C-förmig ist. Auf der der Scharniernase gegenüberliegenden Seite ist die Anpressscheibe 31 mit einer Schraube 43 mit dem Schaftkopf 17 verschraubt. Dies ergibt eine Dreipunkt-Verspannung zwischen den beiden Scharnieren 39,41 und der Schraube 43.

Liegen die Kreiskanten 23 und 37 an der Kugeloberfläche des Anlenkkopfes 25 an, ohne dass Druck auf diesen ausgeübt wird, so ist das Halsteil 21 kugelgelenkig gegenüber dem Schaftkopf 17 verschwenkbar. Zur Fixierung des Anlenkkopfes 21 im Schaftkopf 17 wird die Schraube 43 angezogen. Damit werden die beiden Kreiskanten 23,37 aufeinander zu bewegt. Dabei werden die Kreiskanten 23,37 in die Kugeloberfläche des Anlenkkopfes eingepresst und verformen diesen plastisch. Dadurch wird eine sehr steife Verbindung zwischen dem im Knochen verankerbaren Schaftteil 13 und dem Halsteil 21 erreicht.

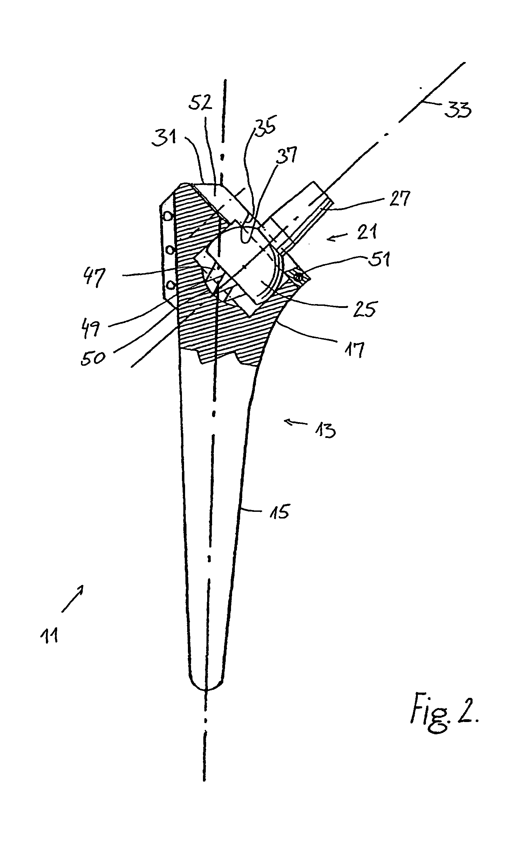

Figur 2 zeigt ein zweites Ausführungsbeispiel einer Schultergelenkprothese 11. Der Anlenkkopf weist einen Kugeloberflächenbereich 45 und drei Spitzkörper 47 auf. Die Spitzen 49 der Spitzkörper 47 weisen denselben Abstand vom Kugelmittelpunkt auf wie die Kugeloberfläche. Die Spitzen 49 sind gegen den Grund der Anlenkmulde 19 gerichtet Im Schwenkbereich der Spitzen 49 besitzt die Anlenkmulde 19 eine Hohlkugeloberfläche 50 mit demselben Radius wie die Kugeloberfläche des Anlenkkopfes 21. An dieser Hohlkugeloberfläche liegen die Spitzen 49 an. Die Hohlkugeloberfläche 50 ist im Grund einer zylindrischen Anlenkmulde 19 ausgebildet. Ein Grosskreis der Kugeloberfläche des Anlenkkopfes 21 liegt an der zylindrischen Mantelfläche der Anlenkmulde 19 an.

Ein Halsfortsatz 27 reicht durch eine Durchtrittöffnung 35 in einer Anpressscheibe 31 hindurch. An ihm wird eine aus einem Set von Kopfkalotten ausgewählte Kopfkalotte befestigt. Die Anpressscheibe 31 ist einseitig mit einer Achse 51 am Schaftkopf 17 angelenkt und gegenüber diesem um die Achse 51 verschwenkbar. Die Anpressscheibe31 ist C-förmig und weist mit der C-Öffnung 52 von der Achse 51 weg. Dadurch ist es möglich, bei aufgeklappter Anpressscheibe 31 den Anlenkkopf 256 zuerst in die Anlenkmulde 19 einzuführen und hernach die Anpressscheibe zurückzuklappen. Mit je einer Schraube in den beiden C-Schenkeln (es ist lediglich die Achse der Schraube dargestellt) ist die Anpressscheibe 31 mit dem Schaftkopf 17 schraubbar. Die Durchtrittöffnung 35 weist hier wie im ersten Ausführungsbeispiel eine zum in der Anlenkmulde19 liegenden Anlenkkopf 25 gerichtete Öffnungskante 37 auf. Mit dieser Öffnungskante 37 wird beim Anziehen der Schraube 43 der Anlenkkopf 25 gegen den Grund der Anlenkmulde 19 gepresst. Dabei graben sich die Spitzen 49 der Spitzkörper 47 in die Hohlkugeloberfläche 50 der Anlenkmulde 19 ein. Je nach Druckverhältnissen und gewählten Materialien gräbt sich auch die Öffnungskante 37 in die Kugeloberfläche des Anlenkkopfes ein.

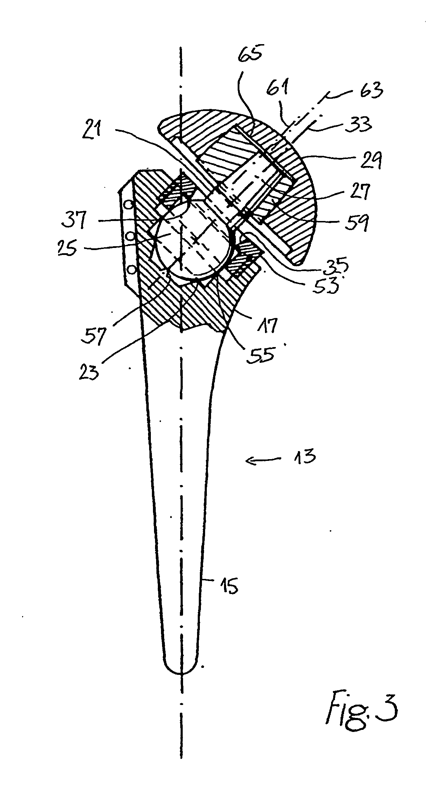

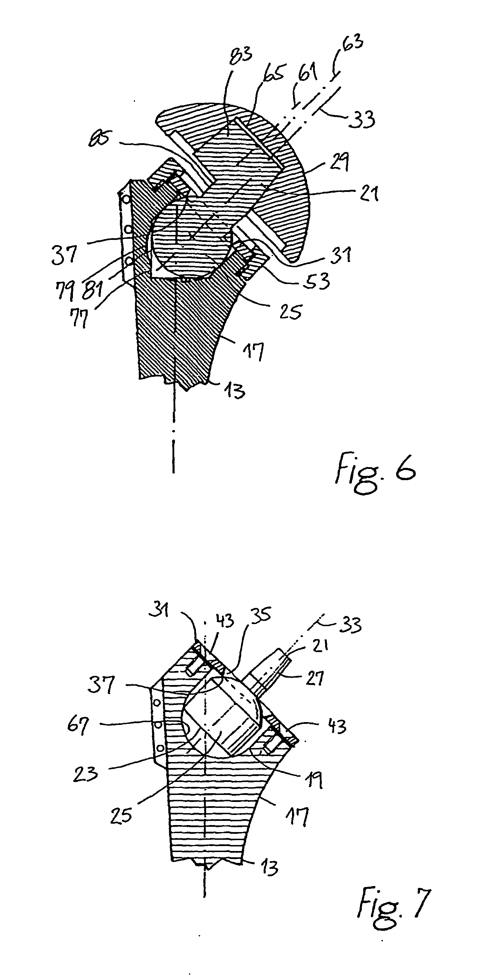

Im in Figur 3 dargestellten dritten Ausführungsbeispiel ist das Pressteil durch eine Überwurfmutter 53 gebildet. Diese ist in ein Gewinde im Rand der Anlenkmulde 19 in die Anlenkmulde 19 einschraubbar. Sie kann auch analog zur Überwurfmutter in den Ausführungsbeispielen gemäss Figur 6 und 7 den Rand der Anlenkmulde 19 umfassen. Hier jedoch liegt die Überwurfmutter auch entlang praktisch eines Grosskreises am kugelförmigen Anlenkkopf 25 an und bildet so eine seitliche Führung für diesen. Diese Führung ist nicht unbedingt notwendig, da die Führung des Anlenkkopfes 25 schon durch die beiden Kreiskanten 23 und 37 erreicht ist. Die Überwurfmutter greift analog zur Anpressplatte 31 der ersten zwei Ausführungsbeispiele mit dem Öffnungsrand 37 der Durchtrittöffnung 35 für den Halsfortsatz 27 am Anlenkkopf 25 an.

Die zylindrisch abgestufte Anlenkmulde 19 weist einen ersten Radius mit dem Gewinde auf. Ein kleinerer zweiter Radius weist beinahe den Radius des Anlenkkopfes 25 auf. Die Mündungskante dieses zweiten Hohlzylinders mit dem zweiten Radius bildet eine erste Kreiskante 55. Der Anlenkkopf wird beim Einpressen in diesen zweiten Hohlzylinder eingepresst, wobei sich zwischen dem Anlenkkopf 25 und der ersten Kreiskante 55 ein Klemmsitz einstellt. Eine zweite Kreiskante 23 mit kleinerem Radius als die erste und eine Körperspitze 57 auf der Achse 33 der Zylinderbohrung am Grund der Anlenkmulde 19 liegen annähernd auf einer Kugeloberfläche mit demselben Radius, wie die Kugel des Anlenkkopfs 25. Beim Einpressen des Anlenkkopfes 25 in die Anlenkmulde 19 kerben diese sich in die Oberfläche des Anlenkkopfes ein.

In Figur 3 ist zudem ein Doppelexzenter zur präzisen Ausrichtung der Kopfkalotte 29 auf die Umrisslinie der Schnittfläche am Knochen dargestellt Der Doppelexzenter setzt sich zusammen aus einem auf den Halsfortsatz 27 aufsteckbaren Exzenterring 59 mit der Achse 61 und einer exzentrisch zur Kalottenachse 63 in der Kopfkalotte 29 angeordneten Ausnehmung 65 zur Aufnahme des Exzenterringes 59. Zwischen Halsfortsatz 29 und Exzenterring 59, bzw. zwischen letzterem und der Ausnehmung 65 ist ein Klemmsitz vorgesehen. Die Trennung von Halsteil 21 und Exzenterring 59 ist deshalb notwendig, weil der Exzenterring 59 nicht durch die Durchtrittöffnung 37 hindurch passt. Wenn der Halsfortsatz 27 in den Anlenkkopf 25 einsetzbar ist, kann der Exzenterring 59 auch einstückig mit dem Halsfortsatz 27 hergestellt sein.

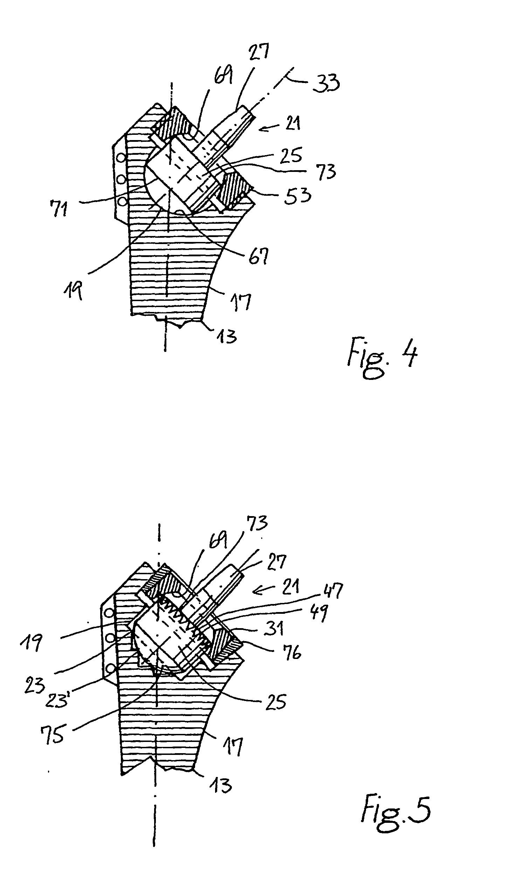

Da die Erfindung unabhängig von der Form des Schaftteils oder Schaftteils 13 ist, ist in den Figuren 4 bis 9 lediglich der Schaftkopf 17 mit Halsteil 21 und Pressteil, z.T. mit. z.T. ohne Exzenter und Kopfkalotte 29 dargestellt.

Figur 4 zeigt ein viertes Ausführungsbeispiel mit einer Anlenkmulde 19 im Schaftkopf 17 mit etwa halbkugeliger Anlenkfläche 67, einer Überwurfmutter 53 mit konkaver, etwa halbkugeliger Anlenkfläche 69 und einem Halsteil 21 mit zylindrischem Anlenkkopf 25. Der zylindrische Anlenkkopf 25 weist zwei Kreiskanten 71,73 auf, von denen eine mit der Anlenkfläche 67 der Anlenkmulde 19 und die andere mit der Anlenkfläche 69 der Überwurfmutter 53 zusammenwirkt. Die Kreiskanten 71,73 liegen auf einer virtuellen Kugeloberfläche mit etwa dem gleichen Radius wie die Kugeloberflächen 67,69 von Anlenkmulde 19 und Überwurfschraube 53. Zur Justierung des Kugelradius der virtuellen Kugeloberfläche genügt es, die Länge des Anlenkkopfes 25 zu verändern. Der Radius der virtuellen Kugel ist vorteilhaft etwas grösser als der Radius der Hohlkugeloberflächen der beiden Anlenkflächen 67,69. Eine genügende Genauigkeit der Übereinstimmung der beiden Kugelradien ist einfach zu erreichen, da die Anlenkflächen 67,69 bzw. die Kreiskanten 71,73 plastisch verformbar sind.

In Figur 5 ist ein fünftes Ausführungsbeispiel dargestellt. Bei diesem ist die Anpressplatte 31 keine Überwurfmutter mit einem Gewinde, sondern hält mit einem Klemmsitz im Schaftkopf 17. Sie weist eine hohlkugelige Anlenkfläche 69 auf, welche mit einer unterbrochen Kreiskante 73 an einem teilweise zylindrischen Anlenkkopf 25 zusammenwirkt. Die Kreiskante 73 ist durch Einschnitte in den Anlenkkopf 25 zu einer Reihe von Körperspitzen 47 aufgelöst, deren Spitzen 49 sich beim Festschlagen der Anlenkscheibe 31 in den Schaftkopf 17 in die Anlenkfläche 69 eingraben. Die Anlenkmulde 19 weist zwei Kreiskanten 23, 23' auf, welche mit einer Kugeloberfläche 75 am Anlenkkopf 25 zusammenwirken. Auch in diesem Ausführungsbeispiel ist die Übereinstimmung der Radien der Kugeloberfläche 75und der Kreiskante 73 am Anlenkkopf 25, mit den Radien von Virtueller Kugeloberfläche, auf der die Kreiskanten 23,23' liegen und der Anlenkfläche 69 an der Anpressscheibe 31 relativ leicht zu erreichen, da die gewünschte Verformung der Berührungsbereiche grössere Toleranzen zulässt, als es eine Verpressung zweier kongruenter Anlenkflächen kann, Die Verklemmung zwischen Schaftteil 13 und Anpressscheibe 31 kann direkt über einen Konus erreicht werden. Besser wird jedoch die Verklemmung der Anpressscheibe 31 wie dargestellt über eine Keilklemmung mit einem z.B. C-förmig die Anpressscheibe umgreifenden Keil 76 erreicht

Das in Figur 6 dargestellte sechste Ausführungsbeispiel weist einen kegelförmigen Grund 77 der Anlenkmulde 19 und einen kugelförmigen Anlenkkopf 25 des Halsteils 21 auf. Die Mantelfläche des Grundes 77 weist Einschnitte 79 in Richtung der Mantellinien durch die Kegelspitze auf. Dadurch ergeben sich zwischen den Einschnitten 79 Kanten 81 quer zu einer Rotationsrichtung um die Achse 33. Die Anpressscheibe 31 ist mit einer Überwurfmutter 53 am Schaftkopf 17 befestigt. Das Halsteil 21 ist einstückig mit einem Exzenter 83 ausgerüstet. Die Anpressscheibe 31 ist C-förmig. Die C-Öffnung weist eine Weite auf, welche es erlaubt, die einen Halsbereich 85 zwischen Anlenkkopf 25 und Exzenter 83 des Halsteils 21 durch diese C-Öffnung hindurch in die Durchtrittöffnung 37 der Anpressscheibe 31 einzuführen. Die Überwurfmutter 53 ist entweder auch C-förmig oder weist einer Öffnungsweite auf, die es erlaubt, die Überwurfmutter 53 über den Exzenter 83 hinab oder über den Anlenkkopf 25 hinauf auf das Halsteil 21 zu schieben. Im letzteren Fall muss zuerst die Überwurfmutter über den Anlenkkopf 25 geschoben und danach die Anpressscheibe 31 zwischen Anlenkkopf 25 und Überwurfmutter 53 eingeschoben werden. Die Anlenkscheibe 31 ist in diesem Beispiel gegenüber der Überwurfmutter 53 verdrehbar. Dies hat den Vorteil, dass beim Anziehen der Überwurfmutter 53 kaum ein Drehmoment auf das Halsteil 21 übertragen wird. Die Öffnungskante 37 wird beim Anziehen der Überwurfmutter 53 gegenüber dem Anlenkkopf 25 nicht verdreht, sondern lediglich gegen diesen gepresst. In nicht festgezogenem Zustand kann das Halsteil 21 um die Halsachse 33 verdreht werden. Dadurch kann der Exzenter 83 in die gewünschte Lage gebracht werden.

Figur 7 zeigt anhand eines siebentes Ausführungsbeispiels eine sehr einfache Variante der Anpressplatte und eine weitere Möglichkeit der Kombination von zylindrischen und Kugelförmigen Körpern und Öffnungen zur Erreichung einer bereits mehrfach ausgeführten kugelgelenluartigen Verbindung zwischen Anlenkmulde und Anlenkkopf bzw. zwischen Schaftkopf 17 und Halsteil 21. In diesem Beispiel weist die Anlenkmulde einen halbkugelförmigen Grund 67 auf, der mit einer Kreiskante 23 eines zylindrischen Anlenkkopfes 25 zusammenwirkt, zur Anpressscheibe 31 hin ist der Anlenkkopf jedoch kugelig geformt, so dass die kreisrunde Öffnungskante 37 der Anpressscheibe 31 am Anlenkkopf in jeder Schwenkstellung desselben gleichermassen anliegt. Die Anlenkscheibe 31 ist mit zwei Schrauben 43 an den Schaftkopf 17 anschraubbar.

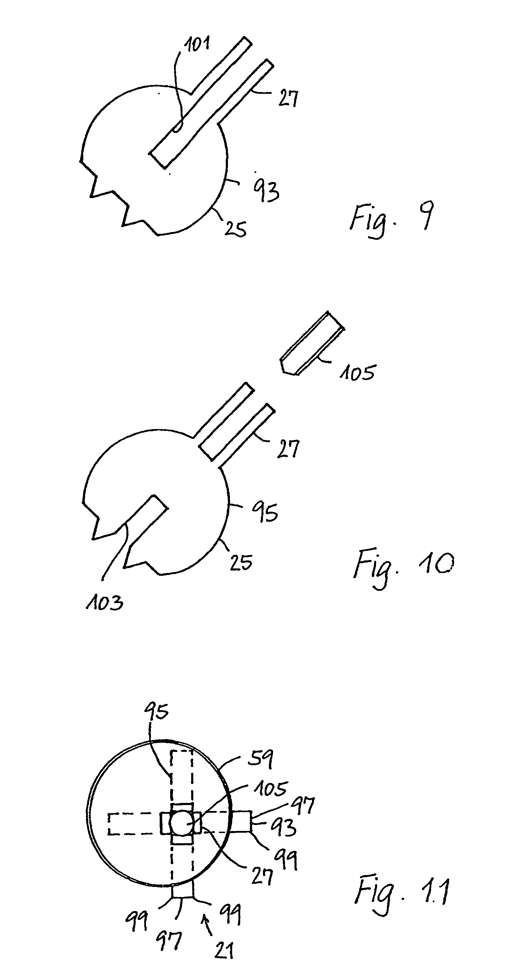

Figur 8 illustriert noch, dass die Erfindung auch ohne Anpressscheibe 31 auskommt Das Halsteil 21 ist in diesem achten Ausführungsbeispiel zylindrisch ausgebildet und weist dank einer Abstufung das Anlenkkopfes 25 zwei Kreiskanten 23,23' auf einer virtuellen Kugeloberfläche auf. Diese Kreiskanten 23,23' arbeiten zusammen mit der Hohlkugeloberfläche der Anlenkmulde 19. Das Halsteil 21 ist nun hohl ausgebildet und eine Schraube 87 sitzt derart in dieser Höhlung 89, dass Ihr Gewindeabschnitt durch eine stirnseitige Öffnung im Halsteil austritt und im Grund der Anlenkmulde einschraubbar ist Am kegelstumpfförmigen Schraubenkopf ist eine Kreiskante 91 ausgebildet, die mit einer Hohlkugeloberfläche am Grund der Höhlung 89 zusammenwirkt Das Halsteil 21 ist einstückig mit dem Exzenter 83 ausgebildet. In festgezogenem Zustand der Schraube 87 sind die Kanten 91, 23, 23' in die Kugeloberflächen eingepresst und verhindern dadurch zuverlässig eine Verschwenkung des Halsteils 21 gegenüber dem Schaftkopf 17. Dann fallen die Kugelmittelpunkte der verschiedenen Kugeloberflächen vorteilhaft praktisch zusammen. Kleinere Abweichungen von einer exakten Übereinstimmung der Kugelmittelpunkte werden jedoch durch die Verformungen kompensiert.

Die Ausführungsbeispiele sind ferner dahingehend abwandelbar, dass die Anlenkmulde im Halsteil und der Anlenkkopf im Schaftkopf ausgebildet ist. Der Halsfortsatz muss mit dem Anlenkkopf nicht einstückig ausgebildet sein, sondern kann als ein im Anlenkkopf festmachbarer Stiel vorgesehen sein. Die Ausbildung des Pressteils ist weitgehend unabhängig von der Ausbildung der Anlenkflächen zwischen Anlenkmulde und Anlenkkopf, so dass die unterschiedlichsten Kombinationen unter den dargestellten und erwähnten Ausführungsvariationen möglich sind.

Weiter ist zu erwähnen, dass z.B. der Anlenkkopf 25 kein Vollkörper sein muss. Er kann beispielsweise wie in Figur 9 bis 11 dargestellt, aus Einzelteilen zusammengesetzt sein. Figur 9 und 10 zeigen die Ansichten von zwei zusammensteckbaren plattenförmigen Teilen 93 und 95. Zusammengesetzt ergeben die Teile 93,95 ein Halsteil 21, das anstelle des Halsteils 21 in Figur 2 eingesetzt werden kann. Die Teile 93,95 sind einfach in der Herstellung. Sie können z.B. mit Laser aus einer Platte geschnitten oder auch gegossen werden. Die Randfläche 97 muss nicht eine Kugeloberfläche sein. Durch die Einpressung in die Anlenkmulde werden die Kanten 99 derart verformt, dass sowohl eine grossflächige Auflage als auch eine sehr gute Verzahnung zwischen Schaftkopf 17 und Halsteil 21 erreicht ist Der Teil 93 in Figur 9 ist mit dem Schlitz 101 über den Schlitz 103 am Teil 95 in Figur 10 hinweg auf dieses Teil 95 aufschiebbar, so dass alle Kanten 99 auf einer gemeinsamen Kugeloberfläche liegen. Im Halsfortsatz 27 ist in beiden Teilen 93,95 eine Nut ausgebildet, in welche eine Schraube 105 einschraubbar ist.

Der Halsfortsatz 27 aus beiden Teilen 93,93 bildet ein Kreuz mit einer zentralen, quadratischen Ausnehmung. Auf dieses Kreuz ist nun der Exzenterring 59 aufsetzbar. Dank der Kreuzform des Halsfortsatzes 27 und einer entsprechenden Ausnehmung im Exzenterring ist eine Verdrehung des Exzenterringes 59 gegenüber dem Halsteil 21 verunmöglicht. Zur Fixation des Exzenterringes 59 auf dem Halsfortsatz 27 und der beiden Teile 93,95 aneinander kann nun die Schraube 105 eingeschraubt werden, was eine Verklemmung zwischen den Teilen 93,95 einerseits und dem Exzenterring 59 andererseits bewirkt. In Figur 11 ist das zusammengesetzte Halsteil 21 mit Exzenterring 59 in einer Aufsicht dargestellt. In dieser Ansicht ist erkennbar, wie die Randflächen 97 der beiden Teile orthogonal zur Plattenebene ausgebildet sind.

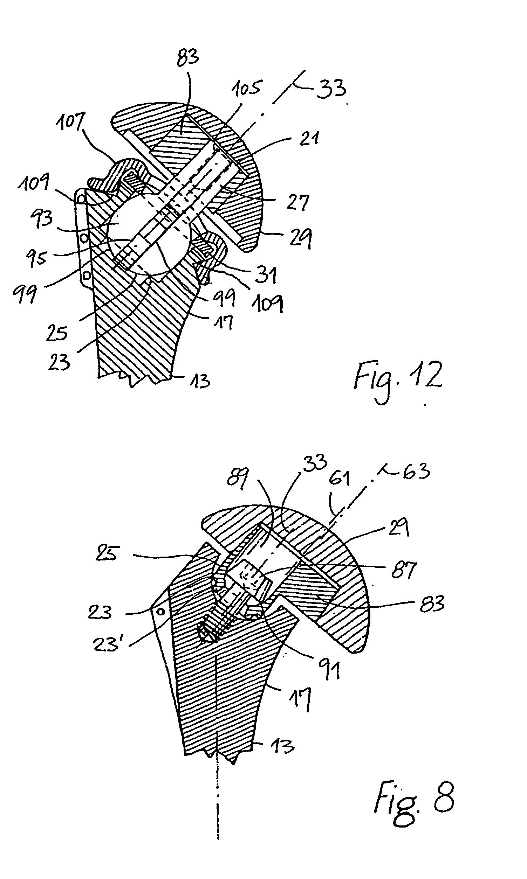

In Figur 12 ist ein zehntes Ausführungsbeispiel dargestellt, in welchem das Halsteil 21 aus zwei Teilen 93,95 zusammengesetzt ist Das Halsteil 21 entspricht bis auf die Form des Anlenkkopfes 25 exakt dem in Figuren 9 bis 11 dargestellten Halsteil 21. Der Schaftkopf 17 ist mit einer zylindrischen Anlenkmulde 19 ausgestattet, in welcher eine Körperkante 23 ausgebildet ist, an der die Kanten 99 des Halsteils 21 anliegen. Das Halsteil 21 ist mit einer Anpressscheibe 31 gegen den Grund der Anlenkmulde 19 gepresst. Die Kraft, mit der die Anpressscheibe 31 gegen den Schaft 13 gepresst wird, ist durch einen Spannbügel 107 auf diese übertragen. Der Spannbügel 107 liegt auf der Aussenseite der Anpressscheibe 31 an und hintergreift einen Hinterschnitt 109 im Schaftkopf 17. Er ist aus einem federnden Material gefertigt, so dass eine relativ grosse Kraft auf die Anpressplatte ausgeübt wird. Der Spannbügel 107 kann auch unmittelbar die Anpressscheibe 31 bilden.

## Patentansprüche

1. Gelenkprothese (11), mit
- einer in einem ersten Knochen verankerbaren künstlichen Gelenkpfanne (12) oder zum gelenkigen Zusammenwirken mit einer natürlichen Gelenkpfanne im ersten Knochen,
- einem in einem zweiten Knochen verankerbaren Schaftteil (13),
- einer Anlenkmulde (19) im Schaftteil (13),
- einem Halsteil (21), das eine Halsachse definiert und einen Anlenkkopf (25) aufweist, welcher Anlenkkopf (25) in der Anlenkmulde (19) angeordnet ist,
- in der Anlenkmulde (19) und am Anlenkkopf (25) derart zusammenwirkende Anlenkflächen, dass das Halsteil (21) kugelgelenkig am Schaftteil angelenkt ist,
- wenigstens einem Pressteil (31,53,87) zum Anpressen des Anlenkkopfes (25) in die Anlenkmulde (19) und
- Mitteln (53,43) zum Verbinden von Pressteil (31,53,87) und Schaftteil (13), sowie
- einer am Halsteil (21) angeordneten Kopfkalotte (29),
**dadurch gekennzeichnet,**
- **dass** eine erste der beiden zusammenwirkenden Anlenkflächen (z.B. 23,23',49,50,57,67,71,75,81,99) wenigstens eine Kante und/ oder Spitze aufweist, und die zweite Anlenkfläche auf einer virtuellen Kugeloberfläche liegt, so dass lediglich ein oder mehrere punktuelle und/ oder linienförmige Berührungsbereiche zwischen der ersten Anlenkfläche und der zweiten Anlenkfläche vorliegen, und
- **dass** die Anlenkflächen bezüglich Material derart ausgebildet sind, dass unter Einwirkung der beim Anpressen auftretenden Druckkräfte wenigstens die zweite Anlenkfläche (z.B. 23,23',49,50,57,67,71,75,81,99) durch die Kante und/ oder Spitze der ersten Anlenkfläche plastisch verformbar ist.

2. Gelenkprothese nach einem der Ansprüche 1 bis 3, **dadurch gekennzeichnet, dass** die zweite Anlenkfläche durch einen glatten oder rauhen Flächenbereich (z.B. 50,67,75) gebildet ist.

3. Gelenkprothese nach Anspruch 1 oder 2, **dadurch gekennzeichnet, dass** die Kanten (z.B. 81) quer zu einer Rotationsrichtung des Halsteils (21) um seine Halsachse (33) liegen.

4. Gelenkprothese nach einem der Ansprüche 1 bis 3, **dadurch gekennzeichnet, dass** der Kugeloberflächenbereich Rillen aufweist.

5. Gelenkprothese nach Anspruch 4, **dadurch gekennzeichnet, dass** die Rillen quer zu einer Rotationsrichtung des Halsteils (21) um seine Halsachse (33) liegen.

6. Gelenkprothese nach einem der Ansprüche 1 bis 5, **dadurch gekennzeichnet, dass** wenigstens eine durchgehende oder unterbrochene Linienberührung zwischen Anlenkkopf (25) und Pressteil (31,53,87) besteht.

7. Gelenkprothese nach einem der Ansprüche 1 bis 6, **dadurch gekennzeichnet, dass** Pressteil (31,53,87) und Schaftteil (13) miteinander verschraubbar sind.

8. Gelenkprothese nach einem der Ansprüche 1 bis 7, **dadurch gekennzeichnet, dass** das Pressteil eine Anpressscheibe (31,53) mit einer Durchtrittöffnung (35) für das Halsteil (21) aufweist.

9. Gelenkprothese nach Anspruch 8, **dadurch gekennzeichnet, dass** die Anpressscheibe (31) ringförmig ist.

10. Gelenkprothese nach Anspruch 9, **dadurch gekennzeichnet, dass** die Anpressscheibe (31) C-förmig ist.

11. Gelenkprothese nach einem der Ansprüche 7 bis 10, **dadurch gekennzeichnet, dass** das Pressteil eine Überwurfmutter (53) ist oder mit einer Überwurfmutter (53) befestigbar ist.

12. Gelenkprothese nach einem der Ansprüche 7 bis 11, **dadurch gekennzeichnet, dass** die Anpressscheibe (31) gegenüber dem Schaftteil (13) um eine Achse durch die Durchtrittöffnung (35) verdrehbar ist

13. Gelenkprothese nach Anspruch 11, allenfalls in Verbindung mit Anspruch 12, **dadurch gekennzeichnet, dass** die Überwurfmutter (53) mit einem Bajonettverschluss mit dem Schaftteil (13) verbindbar ist.

14. Gelenkprothese nach einem der Ansprüche 1 bis 11, **dadurch gekennzeichnet, dass** das Pressteil (31,53,87) gegenüber dem Schaftteil (13) bezüglich einer Achse durch die Durchtrittöffnung (35) unverdrehbar ist.

15. Gelenkprothese nach einem der Ansprüche 7 bis 10, **dadurch gekennzeichnet, dass** die Anpressscheibe (31) durch federnde Spannmittel (107) mit dem Schaftteil (13) verbindbar ist.

16. Gelenkprothese nach einem der Ansprüche 1 bis 8, allenfalls in Verbindung mit einem der Ansprüche 11 oder 12, **dadurch gekennzeichnet, dass** die Anpressscheibe (31) um eine Achse quer zur Halsachse (33) verschwenkbar am Schaftteil (13) befestigt ist.

17. Gelenkprothese nach einem der Ansprüche 1 bis 10, allenfalls in Verbindung mit einem der Ansprüche 15 bis 17, **dadurch gekennzeichnet, dass** die Anpressscheibe (31) über eine Klemmverbindung, z.B. eine Keilklemmung, am Schaftteil (13) festklemmbar ist.

## Claims

1. A joint prosthesis (11), with
- an artificial joint socket (12) for anchoring in a first bone, or for an articulating co-operation with a natural joint socket in a first bone,
- a shaft piece (13) for anchoring in a second bone,
- a joint cavity (19) in the shaft piece (13),
- a collar piece (21) which defines a collar axis and comprises a joint head (25), which joint head (25) is disposed in the joint cavity (19),
- articulation surfaces so co-operating in the joint cavity (19) and at the joint head (25) that the collar piece (21) is articulated on the shaft piece in the manner of a ball joint,
- at least one pressure piece (31, 53, 87) for pressing the joint head (25) into the joint cavity (19) and
- means (53, 43) for connecting the pressure piece (31, 53, 87) and the shaft piece (13), and
- a head cap (29) disposed on the collar piece (21),
**characterized in that**
- a first one of the two co-operating articulation surfaces (e.g. 23, 23', 49, 50, 57, 67, 71, 75, 81, 99) has at least one edge and/or point and the second articulation surface lies on a virtual spherical surface so that only one or more punctiform and/or linear contact zones are present between the first articulation surface and the second articulation surface and
- **in that** the articulation surfaces are so constructed in respect of material that under the action of the pressure forces occurring during pressing into contact at least the second articulation surface (for example 23, 23', 49, 50, 57, 67, 71, 75, 81, 99) is plastically deformable by the edge and/or point of the first articulation surface.

2. A joint prosthesis according to claim 1, **characterized in that** the second articulation surface is formed by a smooth or rough surface zone (for example, 50, 67, 75).

3. A joint prosthesis according to claim 1 or 2, **characterized in that** the edges (e.g. 81) are situated transversely of a direction of rotation of the collar piece (21) about its collar axis (33).

4. A joint prosthesis according to any one of claims 1 to 3, **characterized in that** the spherical surface zone has grooves.

5. A joint prosthesis according to claim 4, **characterized in that** the grooves are situated transversely of a direction of rotation of the collar piece (21) about its collar axis (33).

6. A joint prosthesis according to any one of claims 1 to 5, **characterized in that** at least one continuous or interrupted linear contact exists between the joint head (25) and the pressure piece (31, 53, 87).

7. A joint prosthesis according to any one of claims 1 to 6, **characterized in that** the pressure piece (31, 53, 87) and the shaft piece (13) are adapted to be screwed together.

8. A joint prosthesis according to any one of claims 1 to 7, **characterized in that** the pressure piece comprises a pressure disc (31, 53) with a passage opening (35) for the collar piece (21).

9. A joint prosthesis according to claim 8, **characterized in that** the pressure disc (31) is annular.

10. A joint prosthesis according to claim 9, **characterized in that** the pressure disc (31) is C-shaped.

11. A joint prosthesis according to any one of claims 7 to 10, **characterized in that** the pressure piece is a cap nut (53) or is fixable by a cap nut (53).

12. A joint prosthesis according to any one of claims 7 to 11, **characterized in that** the pressure disc (31) is rotatable relatively to the shaft piece (13) about an axis through the passage opening (35).

13. A joint prosthesis according to claim 11, at least in conjunction with claim 12, **characterized in that** the cap nut (53) is adapted to be connected to the shaft piece (13) by a bayonet fastener.

14. A joint prosthesis according to any one of claims 1 to 11, **characterized in that** the pressure piece (31, 53, 87) is non-rotatable relatively to the shaft piece (13) with respect to an axis through the passage opening (35).

15. A joint prosthesis according to any one of claims 7 to 10, **characterized in that** the pressure disc (31) is adapted to be connected to the shaft piece (13) by resilient clamping means (107).

16. A joint prosthesis according to any one of claims 1 to 8, optionally in conjunction with claim 11 or 12, **characterized in that** the pressure disc (31) is fixed on the shaft piece (13) so as to be pivotable about an axis transversely of the collar axis (33).

17. A joint prosthesis according to any one of claims 1 to 10, optionally in conjunction with any one of claims 15 to 17, **characterized in that** the pressure disc (31) is adapted to be clamped fast on the shaft piece (13) by a clamping connection, for example a wedge clamping.

## Revendications

1. Prothèse d'articulation (11) avec
- une cavité articulaire artificielle (12) pouvant être ancrée dans un premier os ou pour l'action conjointe articulée avec une cavité articulaire naturelle dans le premier os,
- une partie tige (13) pouvant être ancrée dans un second os,
- une cuvette d'articulation (19) dans la partie tige (13),
- une partie de col (21) qui définit un axe de col et qui présente une tête d'articulation (25), laquelle tête d'articulation (25) est placée dans la cuvette d'articulation (19),
- des surfaces d'articulation à action conjointe dans la cuvette d'articulation (19) et sur la tête d'articulation (25) de telle manière que la partie de col (21) est articulée à la manière d'un joint à rotule sur la partie tige,
- au moins une pièce de compression (31, 53, 87) pour presser la tête d'articulation (25) dans la cuvette d'articulation (19) et
- des moyens (53, 43) pour relier la pièce de compression (31, 53, 87) et la partie tige (13) ainsi
- qu'une calotte de tête (29) placée sur la partie de col (21),
**caractérisée en ce**
- **qu'**une première des deux surfaces d'articulation à action conjointe (par exemple 23, 23', 49, 50, 57, 67, 71, 75, 81, 99) présente au moins une arête et/ou pointe et la seconde surface d'articulation se trouve sur une surface sphérique virtuelle si bien qu'il n'y a qu'une ou plusieurs zones de contact ponctuelles et/ou en forme de ligne entre la première surface d'articulation et la seconde surface d'articulation et
- **que** les surfaces d'articulation sont configurées pour ce qui est du matériau de telle manière que, sous l'effet des forces de pression qui interviennent lors de la pression, au moins la seconde surface d'articulation (par exemple 23, 23', 49, 50, 57, 67, 71, 75, 81, 99) peut être déformée plastiquement par l'arête et/ou la pointe de la première surface d'articulation.

2. Prothèse d'articulation selon l'une des revendications 1 à 3, **caractérisée en ce que** la seconde surface d'articulation est formée par une zone de surface lisse ou rugueuse (par exemple 50, 67, 75).

3. Prothèse d'articulation selon la revendication 1 ou 2, **caractérisée en ce que** les arêtes (par exemple 81) se situent transversalement par rapport à un sens de rotation de la partie de col (21) autour de son axe de rotation (33).

4. Prothèse d'articulation selon l'une des revendications 1 à 3, **caractérisée en ce que** la zone de surface sphérique présente des rainures.

5. Prothèse d'articulation selon la revendication 4, **caractérisée en ce que** les rainures se situent transversalement par rapport à un sens de rotation de la partie de col (21) autour de son axe de col (33).

6. Prothèse d'articulation selon l'une des revendications 1 à 5, **caractérisée en ce qu'**il y a au moins un contact en ligne continue ou interrompue entre la tête d'articulation (25) et la pièce de compression (31, 53, 87).

7. Prothèse d'articulation selon l'une des revendications 1 à 6, **caractérisée en ce que** la pièce de compression (31, 53, 87) et la partie tige (13) peuvent être vissées l'une à l'autre.

8. Prothèse d'articulation selon l'une des revendications 1 à 7, **caractérisée en ce que** la pièce de compression présente un disque de compression (31, 53) avec une ouverture de passage (35) pour la partie de col (21).

9. Prothèse d'articulation selon la revendication 8, **caractérisée en ce que** le disque de compression (31) est de forme annulaire.

10. Prothèse d'articulation selon la revendication 8, **caractérisée en ce que** le disque de compression (31) est en forme de C.

11. Prothèse d'articulation selon l'une des revendications 7 à 10, **caractérisée en ce que** la pièce de compression est un écrou-raccord (53) ou peut être fixée avec un écrou-raccord (53).

12. Prothèse d'articulation selon l'une des revendications 7 à 11, **caractérisée en ce que** le disque de compression (31) est rotatif par rapport à la partie tige (13) autour d'un axe à travers l'ouverture de passage (35).

13. Prothèse d'articulation selon la revendication 11, tout au moins en relation avec la revendication 12, **caractérisée en ce que** l'écrou-raccord (53) peut être relié à la partie tige (13) avec une fermeture à baïonnette.

14. Prothèse d'articulation selon l'une des revendications 1 à 11, **caractérisée en ce que** la pièce de compression (31, 53, 87) n'est pas rotative par rapport à la partie tige (13) par rapport à un axe à travers l'ouverture de passage (35).

15. Prothèse d'articulation selon l'une des revendications 7 à 10, **caractérisée en ce que** le disque de compression (31) peut être relié à la partie tige (13) par des moyens de serrage élastiques (107).

16. Prothèse d'articulation selon l'une des revendications 1 à 8, tout au moins en relation avec l'une des revendications 11 ou 12, **caractérisée en ce que** le disque de compression (31) est fixé sur la partie tige (13) en étant pivotant autour d'un axe transversalement par rapport à l'axe du col (33).

17. Prothèse d'articulation selon l'une des revendications 1 à 10, tout au moins en relation avec au moins l'une des revendications 15 à 17, **caractérisée en ce que** le disque de compression (31) peut être bloqué sur la partie tige (13) par une jonction par serrage, par exemple par un serrage de clavette.
